# DEMANDE DE BREVET EUROPEEN

(11) **EP 1 739 073 A1**
(43) Date de publication de la demande: **03.01.2007**
(21) Numéro de dépôt: 06291029.4
(22) Date de dépôt: 23.06.2006
(51) Int. Cl.: C07C 211/51, C07C 215/70, C07C 245/08, C07D 209/44, C07D 217/00, C07D 307/87, C07D 311/76, A61K 8/41, A61K 8/49, A61Q 5/10

(54) **Nouvelles para-phénylènediamines primaires 2,3 disubtituées et leur utilisation en teinture d'oxydation des fibres kératiniques**

(30) Priorité: 28.06.2005 FR 0506566
(71) Demandeur: L'Oreal, 75008 Paris (FR)
(72) Inventeur: Ramos-Stanbury, Laure, F-92330 Sceaux (FR)
(74) Mandataire: Zapalowicz, Francis

(57) **Abrégé**

L'invention concerne des composés para-phénylènediamine de formule (I), leurs sels et solvates : 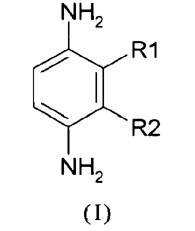 dans laquelle R₁ et R₂ forment ensemble avec les atomes de carbone auxquels ils sont rattachés un cycle à 4, 5, 6 ou 7 chaînons, caractérisé en ce que le cycle est soit constitué d'atomes de carbone, soit constitué d'atomes de carbone et d'un ou deux atomes choisis parmi l'oxygène et l'azote, lorsque le cycle comprend deux atomes choisis parmi l'oxygène et l'azote alors ces deux atomes sont non adjacents, le cycle étant non substitué ou substitué.

## Description

La présente invention est relative à de nouvelles para-phénylènediamines primaires 2,3-disubstituées, à une composition pour la teinture d'oxydation des fibres kératiniques, et en particulier des fibres kératiniques humaines telles que les cheveux, comprenant au moins, comme base d'oxydation, une para-phénylènediamine primaire 2,3-disubstituée, et aux procédés de teinture d'oxydation la mettant en oeuvre.

Il est connu de teindre les fibres kératiniques et en particulier les cheveux humains avec des compositions tinctoriales contenant des précurseurs de colorant d'oxydation, en particulier des ortho- ou para-phénylènediamines, des ortho- ou para-aminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire à un certain nombre d'exigences. Ainsi, elle doit être sans inconvénient sur le plan toxicologique, elle doit permettre d'obtenir des nuances dans l'intensité souhaitée, présenter une bonne tenue aux agents extérieurs (lumière, intempéries, lavage, ondulation permanente, transpiration, frottements).

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui peut être en effet différemment sensibilisée (c'est-à-dire abîmée) entre sa pointe et sa racine. Ils doivent également présenter une bonne stabilité chimique dans les formulations. Ils doivent présenter un bon profil toxicologique.

De plus, pour un certain nombre d'applications, on recherche des colorants donnant sur les cheveux des nuances chromatiques.

Or, la demanderesse vient maintenant de découvrir, de façon avantageuse et surprenante, qu'il était possible d'obtenir des teintures, capables de conduire à des colorations puissantes, peu sélectives, et présentant d'excellentes propriétés de résistance aux diverses agressions que peuvent subir les fibres kératiniques en utilisant à titre de base d'oxydation les para-phénylènediamines primaires 2,3-disubstituées de formule (I) ci-après ou leurs sels ou solvates physiologiquement acceptables.

La présente invention a donc pour premier objet les nouvelles para-phénylènediamines primaires 2,3-disubstituées de formule (I) suivante : 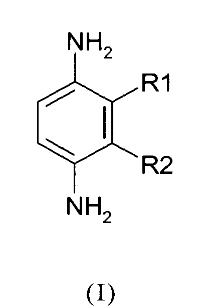 dans laquelle R₁ et R₂ forment ensemble avec les atomes de carbone auxquels ils sont rattachés un cycle à 4, 5, 6 ou 7 chaînons, caractérisé en ce que le cycle est soit constitué d'atomes de carbone, soit constitué d'atomes de carbone et d'un ou deux atomes choisis parmi l'oxygène ou l'azote, lorsque le cycle comprend deux atomes choisis parmi l'oxygène ou l'azote alors ces deux atomes sont non adjacents, le cycle pouvant être substitué notamment par un ou plusieurs radicaux choisis parmi les radicaux alkyle en C₁-C₆, hydroxyle (-OH), alcoxy en C₁-C₆, amino (-NH₂), (mono ou dialkyle) amino en C₁-C₆ ou aminoalkyle en C₁-C₆,
à l'exception des composés suivants :

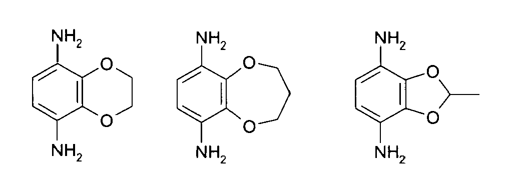

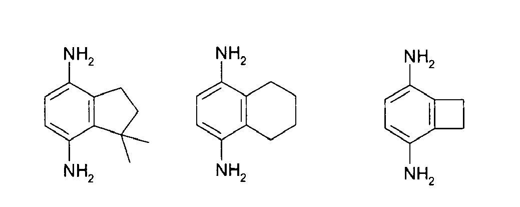

L'invention a également pour objet les solvates et sels d'acides organiques ou minéraux physiologiquement acceptables des composés de formule (I).

D'une manière générale, les sels d'addition utilisables sont notamment choisis parmi les sels d'addition avec un acide, tels que l'acide chlorhydrique, l'acide bromhydrique, l'acide sulfurique, l'acide citrique, l'acide succinique, l'acide tartrique, l'acide lactique, l'acide méthanesulfonique, l'acide para-toluènesulfonique, l'acide benzènesulfonique, l'acide phosphorique et l'acide acétique.

Ils peuvent aussi être sous forme de solvates par exemple un hydrate ou un solvate d'alcool linéaire ou ramifié tel que l'éthanol ou l'isopropanol.

Lorsque le cycle formé par R₁ et R₂ contient un ou deux atomes d'azote alors le cycle peut comprendre un radical divalent azoté -NH- ou -N(alkyl en C₁-C₄)-.

Selon un premier mode préféré, R₁ et R₂ forment ensemble avec les atomes de carbone auxquels ils sont rattachés un cycle à 5 ou 6 chaînons, de manière encore préférée un cycle soit constitué d'atomes de carbone, soit constitué d'atomes de carbone et d'un atome choisi parmi l'oxygène ou un radical azoté (-NH-) ou -NMe-.
De manière encore plus préférée, R₁ et R₂ forment ensemble avec les atomes de carbone auxquels ils sont rattachés un cycle à 5 ou 6 chaînons soit constitué d'atomes de carbone, soit constitué d'atomes de carbone et d'un radical azoté (-NH-) ou -NMe-.

Selon un deuxième mode, le cycle formé par R₁ et R₂ est substitué par 1, 2 ou 3 radicaux identiques ou non choisis parmi les radicaux alkyle en C₁-C₃, hydroxyle (-OH), amino (-NH₂) ou aminoalkyle en C₁-C₃. A titre d'exemples, on peut citer les radicaux amino, hydroxyle, méthyle, éthyle, isopropyle, n-propyle, aminométhyle.

De manière encore préférée, le cycle est substitué par 1 ou 2, de préférence 1, radicaux choisis parmi les radicaux amino, hydroxyle, propyle, aminométhyle, méthyle.

Selon un mode de réalisation particulier, le cycle formé par R₁ et R₂ est un cycle carboné comprenant de préférence 5 à 6 chaînons

A titre de para-phénylènediamines de formule (I) préférées, on peut citer les composés suivants ainsi que leurs sels et/ou solvates.

| | |
|---|---|
| 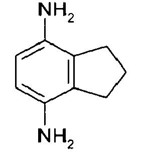 | Indan-4,7-diamine |
| 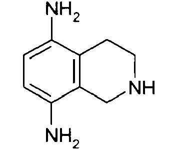 | 1,2,3,4-Tétrahydro-isoquinoline-5,8-diamine |
| 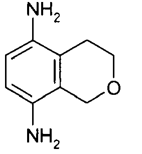 | Isochroman-5,8-diamine |
| 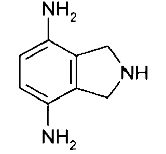 | 2,3-Dihydro-1H-isoindole-4,7-diamine |
| 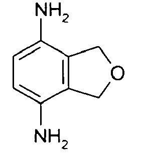 | 1,3-Dihydro-isobenzofuran-4,7-diamine |
| 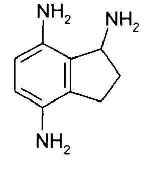 | Indan-1,4,7-triamine |
| 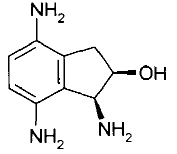 | 1,4,7-Triamino-indan-2-ol |
| 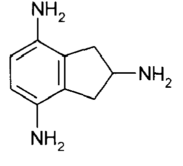 | Indan-2,4,7-triamine |
| 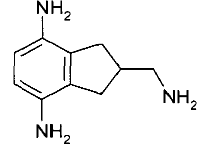 | 2-Aminométhyl-indan-4,7-diamine |
| 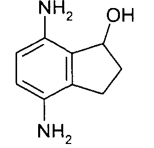 | 4,7-Diamino-indan-1-ol |
| 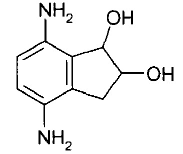 | 4,7-Diamino-indan-1,2-diol |
| 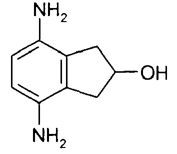 | 4,7-Diamino-indan-2-ol |
| 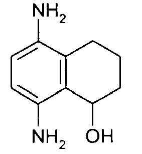 | 5,8-Diamino-1,2,3,4-tétrahydro-naphthalèn-1-ol |
| 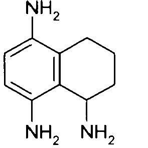 | 5,6,7,8-Tétrahydro-naphthalène-1,4, 5-triamine |
| 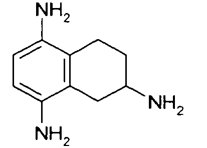 | 5,6,7,8-Tétrahydro-naphthalène-1,4, 6-triamine |
| 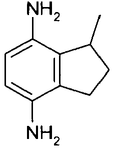 | 1-Méthyl-indan-4,7-diamine |
| 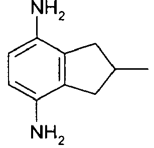 | 2-Méthyl-indan-4,7-diamine |
| 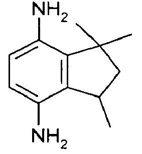 | 1,1,3-Triméthyl-indan-4,7-diamine |
| 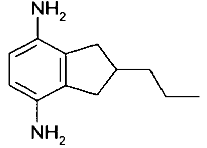 | 2-Propyl-indan-4,7-diamine |
| 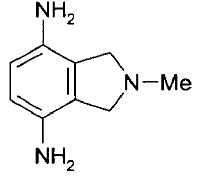 | 2-méthylisoindoline-4,7-diamine |

Les para-phénylènediamines primaires 2,3-substituées de formule (I) plus particulièrement préférées sont :
l'indan-4,7-diamine, le 1,2,3,4-tétrahydro-isoquinoline-5,8-diamine, le 2,3-dihydro-1 H-isoindole-4,7-diamine, le 2-méthylisoindoline-4,7-diamine, l'indan-2,4,7-triamine, le 2-aminométhyl-indan-4,7-diamine, le 4,7-diamino-indan-2-ol, la 2-méthyl-indan-4,7-diamine et le 2-propyl-indan-4,7-diamine ou l'un de leurs sels ou solvates physiologiquement acceptables.

La présente demande vise également un procédé de synthèse d'un composé para-phénylènediamine de formule (I) qui comprend une étape de réduction du composé intermédiaire de formule (II) correspondant : 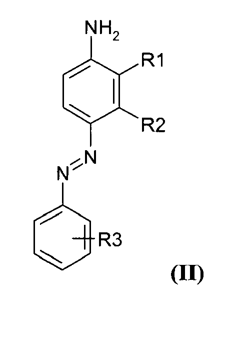 dans laquelle R₁ et R₂ forment ensemble avec les atomes de carbone auxquels ils sont rattachés un cycle à 4, 5, 6 ou 7 chaînons, caractérisé en ce que le cycle est soit constitué d'atomes de carbone, soit constitué d'atomes de carbone et d'un ou deux atomes identiques ou non choisis parmi l'oxygène ou l'azote, lorsque le cycle comprend deux atomes choisis parmi l'oxygène ou l'azote alors ces deux atomes sont non adjacents, le cycle pouvant être substitué notamment par un ou plusieurs radicaux identiques ou non choisis parmi les radicaux alkyle linéaires ou ramifiés en C₁-C₆, hydroxyle (-OH), alcoxy en C₁-C₆, amino (-NH₂), (mono ou dialkyle) amino en C₁-C₆, ou aminoalkyle en C₁-C₆ ; R₃ est choisi dans le groupe formé par un atome d'hydrogène, un groupement sulfonique, les radicaux alkyle linéaires ou ramifiés en C₁-C₄,
à l'exception des composés suivants :

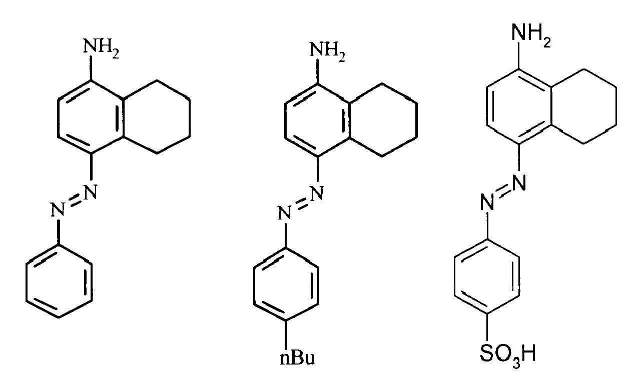

Au sens de la présente demande, « nBu » signifie n-butyle Comme précédemment, dans les formules ci-dessus, lorsque le cycle formé par R₁ et R₂ contient un ou deux atomes d'azote alors le cycle peut comprendre un radical divalent azoté -NH- ou -N(alkyl en C₁-C₄), en particulier -NMe-.

La présente demande vise aussi les composés intermédiaires de formule (II) 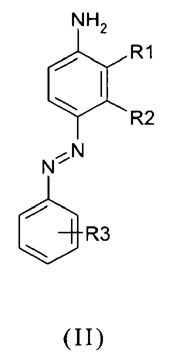 dans laquelle R₁ et R₂ forment ensemble avec les atomes de carbone auxquels ils sont rattachés un cycle à 4, 5, 6 ou 7 chaînons, caractérisé en ce que le cycle est soit constitué d'atomes de carbone, soit constitué d'atomes de carbone et d'un ou deux atomes identiques ou non choisis parmi l'oxygène ou l'azote, lorsque le cycle comprend deux atomes choisis parmi l'oxygène ou l'azote, alors ces deux atomes sont non adjacents, le cycle étant éventuellement substitué notamment par un ou plusieurs radicaux identiques ou non choisis parmi les radicaux alkyles linéaires ou ramifiés en C₁-C₆, hydroxyle (-OH), alcoxy en C₁-C₆, amino (-NH₂), (mono ou dialkyle) amino en C₁-C₆, ou aminoalkyle en C₁-C₆ ; R₃ est choisi dans le groupe formé par un atome d'hydrogène, un groupement sulfonique, les radicaux alkyles linéaires ou ramifiés en C₁-C₄,
à l'exception des composés suivants : 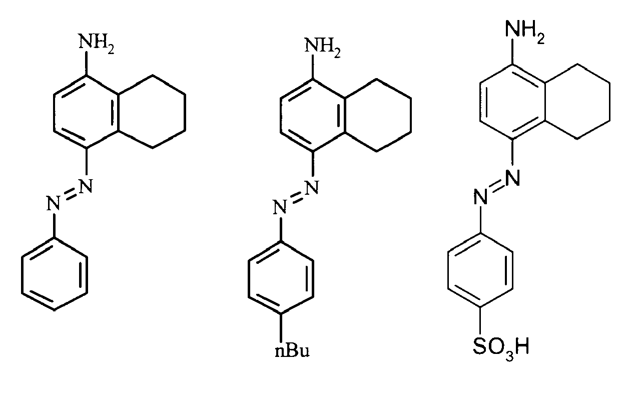 Comme précédemment, dans les formules ci-dessus, lorsque le cycle formé par R₁ et R₂ contient un ou deux atomes d'azote alors le cycle peut comprendre un radical divalent azoté -NH- ou -N(alkyl en C₁-C₄), en particulier ―NMe-.

L'invention a également pour objet une composition pour la teinture d'oxydation des fibres kératiniques, et en particulier des fibres kératiniques humaines telles que les cheveux, caractérisée par le fait qu'elle contient, dans un milieu approprié pour la teinture, au moins un composé para-phénylènediamine de formule (I), ses sels et solvates: 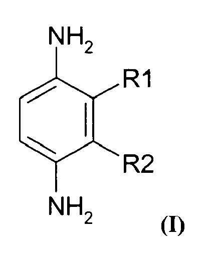 dans laquelle R₁ et R₂ forment ensemble avec les atomes de carbone auxquels ils sont rattachés un cycle à 4, 5, 6 ou 7 chaînons, caractérisé en ce que le cycle est soit constitué d'atomes de carbone, soit constitué d'atomes de carbone et d'un ou deux atomes identiques ou non choisis parmi l'oxygène ou l'azote, lorsque le cycle comprend deux atomes choisis parmi l'oxygène ou l'azote alors ces deux atomes sont non adjacents, le cycle étant éventuellement substitué notamment par un ou plusieurs radicaux identiques ou non choisis parmi les radicaux alkyle en C₁-C₆, hydroxyle (-OH), alcoxy en C₁-C₆, amino (-NH₂), (mono ou dialkyle) amino en C₁-C₆ ou aminoalkyle en C₁-C₆,
à l'exception des composés suivants :

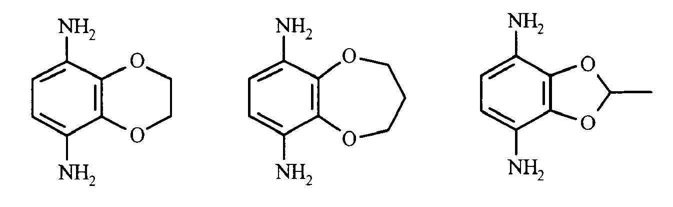

Comme précédemment, dans les formules ci-dessus, lorsque le cycle formé par R₁ et R₂ contient un ou deux atomes d'azote alors le cycle peut comprendre un radical divalent azoté -NH- ou -N(alkyl en C1-C4), en particulier -NMe-.

Les colorations obtenues avec la composition de teinture d'oxydation conforme à l'invention sont puissantes. Elles présentent de plus d'excellentes propriétés de résistance vis-à-vis de l'action des différents agents extérieurs tels que la lumière, les intempéries, le lavage, les ondulations permanentes, la transpiration, les frottements.

Un cinquième objet de la présente demande vise un procédé de teinture d'oxydation des fibres kératiniques mettant en oeuvre une telle composition tinctoriale.

D'autres caractéristiques, aspects, objets et avantages de la présente invention apparaîtront encore plus clairement à la lecture de la description et des exemples qui suivent.

A titre d'exemples de para-phénylènediamines primaires 2,3-substituées de formule (I) utilisables dans les compositions pour la teinture d'oxydation selon l'invention, on peut citer les composés suivants ainsi que leurs sels et/ou solvates :

| | |
|---|---|
| 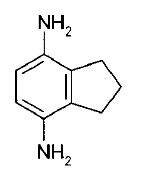 | Indan-4,7-diamine |
| 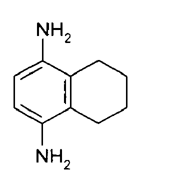 | 5,6,7,8-Tétrahydro-naphtalène-1,4-diamine |
| 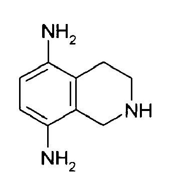 | 1,2,3,4-Tétrahydro-isoquinoline-5,8-diamine |
| 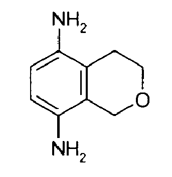 | Isochroman-5,8-diamine |
| 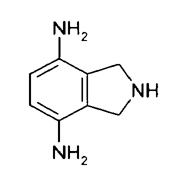 | 2,3-Dihydro-1H-isoindole-4,7-diamine |
| 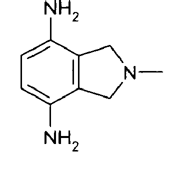 | 2-Méthylisoindoline-4,7-diamine |
| 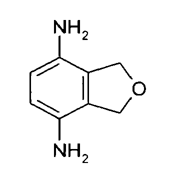 | 1,3-Dihydro-isobenzofuran-4,7-diamine |
| 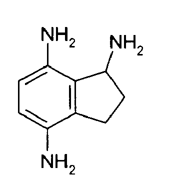 | Indan-1,4,7-triamine |
| 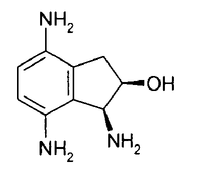 | 1,4,7-Triamino-indan-2-ol |
| 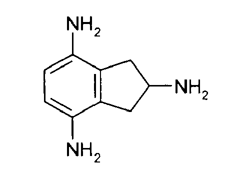 | Indan-2,4,7-triamine |
| 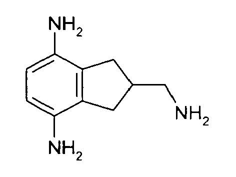 | 2-Aminométhyl-indan-4,7-diamine |
| 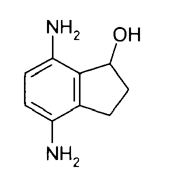 | 4,7-Diamino-indan-1-ol |
| 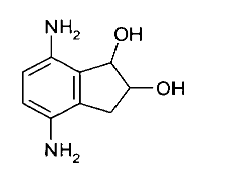 | 4,7-Diamino-indan-1,2-diol |
| 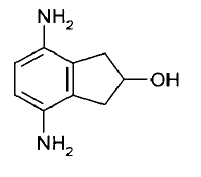 | 4,7-Diamino-indan-2-ol |
| 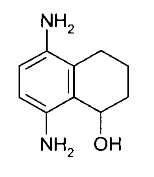 | 5,8-Diamino-1,2,3,4-tétrahydro-naphtalèn-1-ol |
| 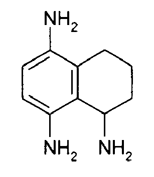 | 5,6,7,8-Tétrahydro-naphtalène-1,4,5-triamine |
| 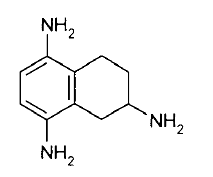 | 5,6,7,8-Tétrahydro-naphtalène-1,4,6-triamine |
| 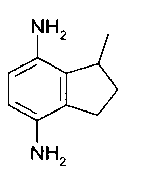 | 1-Méthyl-indan-4,7-diamine |
| 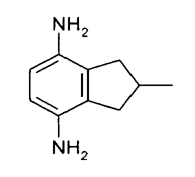 | 2-Méthyl-indan-4,7-diamine |
| 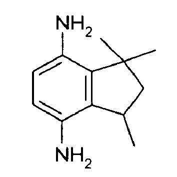 | 1,1,3-Triméthyl-indan-4,7-diamine |
| 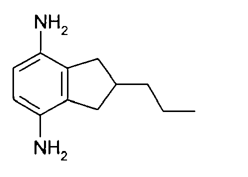 | 2-Propyl-indan-4,7-diamine |

Les para-phénylènediamines primaires 2,3-substituées de formule (I) plus particulièrement préférées utilisables dans les compositions pour la teinture d'oxydation sont :
l'indan-4,7-diamine, le 5,6,7,8-tétrahydro-naphtalène-1,4-diamine, le 1,2,3,4-tétrahydro-isoquinoline-5,8-diamine, le 2,3-dihydro-1H-isoindole-4,7-diamine, le 2-méthylisoindoline-4,7-diamine, l'indan-2,4,7-triamine, le 2-aminométhyl-indan-4,7-diamine, le 4,7-diamino-indan-2-ol, la 2-méthyl-indan-4,7-diamine et le 2-propyl-indan-4,7-diamine ou l'un de leurs sels ou solvates physiologiquement acceptables.

Les para-phénylènediamines primaires 2,3-substituées de formule (I) selon l'invention sont préparés par exemple selon la méthode générale de préparation suivante : 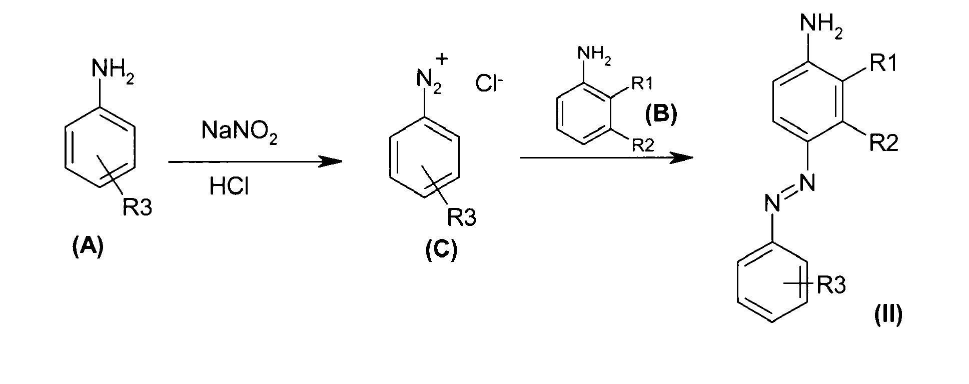 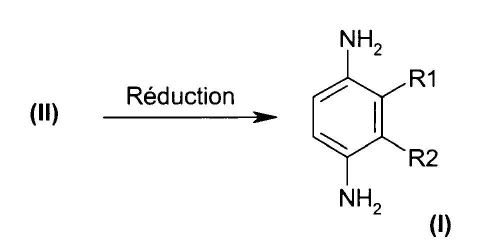 R₁, R₂ et R₃ ayant les significations précédemment énoncées.

La première étape de la synthèse est la formation d'un azoïque (II) par réaction d'un sel de diazonium (C) avec une aniline (B) selon des méthodes bien connues (Hegarty, in Patai The chemistry of Diazonium and Diazo Group, pt.2 ; Wiley : New York, 1978. La deuxième étape permettant d'accéder aux composés (I) est une étape de réduction classique, par exemple en effectuant une réaction d'hydrogénation par catalyse hétérogène en présence, par exemple de de Pd/C, ou encore en effectuant une réaction de réduction par le bisulfite de sodium, certains métaux (notamment le zinc) ou les boranes (Advanced Organic Chemistry, 4th edition, 1992, J. MARCH, WILEY Interscience ; Rreduction in Organic Chemistry, M. Hudlicky, 1983, Ellis Honwood series Chemical Science).

Un composé para-phénylènediamine de formule (I) est obtenu selon un procédé de synthèse qui comprend une étape de réduction du composé intermédiaire de formule (II) correspondant. Par « composé intermédiaire de formule (II) correspondant » on entend que le radical R₁ dudit « composé intermédiaire de formule (II) correspondant » est le même que le radical R₁ du composé de formule (I) et le radical R₂ dudit « composé intermédiaire de formule (II) correspondant » est le même que le radical R₂ du composé de formule (I).

La composition tinctoriale selon l'invention contient notamment de 0,001 à 10% en poids, de préférence de 0,05 à 6% en poids, et encore plus préférentiellement de 0,1 à 3% en poids, d'au moins une para-phénylènediamine primaire 2,3-substituée de formule (I) ou ses sels ou solvates.

La composition tinctoriale conforme à l'invention peut aussi contenir, en plus du (ou des) para-phénylènediamines primaires 2,3-substituées définie(s) ci-dessus, au moins une base d'oxydation additionnelle qui peut être choisie parmi les bases d'oxydation classiquement utilisées en teinture d'oxydation et parmi lesquelles on peut notamment citer les para-phénylènediamines additionnelles différentes des para-phénylènes primaires 2,3-substituées de formule (I), les bis-phénylalkylènediamines, les para-aminophénols, les ortho-aminophénols et des bases hérétocycliques.

Parmi les para-phénylènediamines additionnelles, on peut plus particulièrement citer à titre d'exemple, la para-phénylènediamine, la para-toluylènediamine, la 2,6-diméthyl para-phénylènediamine, la 2-β-hydroxyéthyl para-phénylènediamine, la 2-n-propyl para-phénylènediamine, la 2-isopropyl para-phénylènediamine, la N-(β-hydroxypropyl) para-phénylènediamine, la N,N-bis-(β-hydroxyéthyl) para-phénylènediamine, la 4-amino N-(β-méthoxyéthyl) aniline, les para-phénylènediamines décrites dans la demande de brevet français FR 2630438, et leurs sels d'addition.

Parmi les bis-phénylalkylènediamines, on peut plus particulièrement citer à titre d'exemple, le N,N'-bis-(β-hydroxyéthyl) N,N'-bis-(4'-aminophényl) 1,3-diaminopropanol, la N,N'-bis-(β-hydroxyéthyl) N,N'-bis-(4'-aminophényl) éthylène-diamine, la N,N'-bis-(4-amino phényl) tétra-méthylènediamine, la N,N'-bis-(β-hydroxyéthyl), N,N'-bis-(4-aminophényl) tétra-méthylènediamine, la N,N'-bis-(4-méthylaminophényl) tétra-méthylène-diamine, la N,N'-bis-(éthyl) N,N'-bis-(4'-amino, 3'-méthylphényl) éthylènediamine, et leurs sels d'addition.

Parmi les para-aminophénols, on peut plus particulièrement citer à titre d'exemple, le para-aminophénol, le 4-amino 3-méthyl phénol, le 4-amino 3- fluoro phénol, le 4-amino 3-hydroxy-méthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition.

Parmi les ortho-aminophénols, on peut plus particulièrement citer à titre d'exemple, le 2-amino phénol, le 2-amino 5-méthyl phénol, le 2-amino 6-méthylphénol, le 5-acétamido 2-amino phénol, le 5-[(2-hydroxyethyl)amino]-2-methylphenol et leurs sels d'addition.

Parmi les bases hétérocycliques, on peut plus particulièrement citer à titre d'exemple, les dérivés pyridiniques, les dérivés pyrimidiniques, les dérivés pyrazoliques, et leurs sels d'addition.

Lorsqu'elles sont utilisées, ces bases d'oxydation additionnelles représentent de préférence de 0,0005 à 12% en poids du poids total de la composition tinctoriale, et encore plus préférentiellement de 0,005 à 6% en poids de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les méta-phénylènediamines, les méta-aminophénols, les méta-diphénols, les dérivés mono ou polyhydroxylé du naphtalène et les coupleurs hétérocycliques tels que par exemple les dérivés indoliques ou pyridiniques et leurs sels d'addition.

Ces coupleurs sont plus particulièrement choisis parmi le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Lorsqu'ils sont présents, ces coupleurs représentent notamment de 0,0001 à 10% du poids total de la composition tinctoriale, de préférence de 0,005 à 5% en poids, et encore plus préférentiellement de 0,1 à 3% de ce poids.

D'une manière générale, les sels d'addition avec un acide utilisables dans le cadre des compositions tinctoriales de l'invention (bases d'oxydation et coupleurs) sont notamment choisis parmi les sels des acides suivants : chlorhydrique, bromhydrique, sulfurique, acétique, lactique, tartrique, citrique, méthanesulfonique ou succinique.

Le milieu approprié pour la teinture (ou support) utilisé selon l'invention est constitué par de l'eau ou par un mélange d'eau et d'au moins un solvant organique choisi parmi les alcanols inférieurs en C₁-C₄, les polyols et éthers de polyols, les alcools aromatiques, et leurs mélanges.

La composition tinctoriale selon l'invention peut également contenir divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, des agents antioxydants, des agents réducteurs, des filtres solaires, des agents de pénétration, des agents séquestrants, des parfums, des tampons, des agents dispersants, des agents de conditionnement tels que par exemple des silicones, des agents filmogènes, des agents conservateurs, des agents opacifiants.

Le pH de la composition tinctoriale selon l'invention est compris entre 3 et 12.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant. La composition tinctoriale peut éventuellement contenir des catalyseurs d'oxydation, afin d'accélérer le processus d'oxydation.

Selon une première forme de mise en oeuvre du procédé de l'invention, la coloration des fibres peut être effectuée sans addition d'un agent oxydant, au seul contact de l'oxygène de l'air.

Selon une deuxième forme de mise en oeuvre du procédé de l'invention, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes, de préférence 5 à 30 minutes, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans toutefois en limiter la portée.

### EXEMPLES

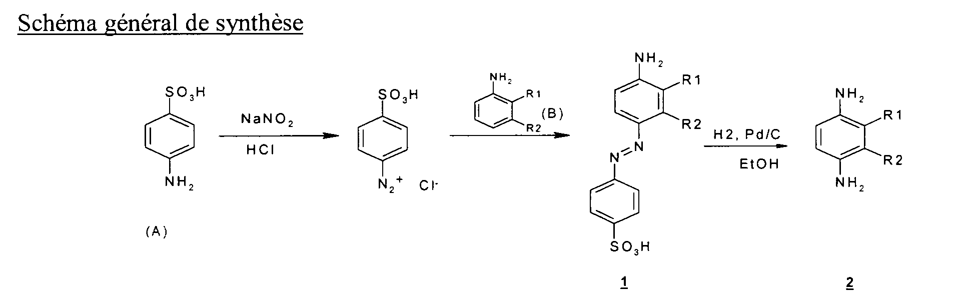

### Synthèse des dérivés d'acide diazénylbenzènesulfonigue (composés 1 selon le schéma précédent)

On prépare une solution de nitrite de sodium dans l'eau qui est additionnée à froid à l'acide sulfanilique dissout dans l'eau (couleur orange). La température est maintenue inférieure à 10 °C.
La solution obtenue est immédiatement versée dans un mélange d'acide chlorhydrique concentré et de glace.
Le sel de diazonium froid est additionné à l'aniline (B) en solution dans HCl 0,5 N (une couleur orange sang apparaît) le précipite formé est filtré et lavé une fois à l'eau.

### 1a) Acide 4-[(E)-(5-amino-1,2,3,4-tétrahydroisoquinolin-8-yl)diazényl]-benzènesulfonique

amine (B): 1,2,3,4 tétrahydro-5-aminosoquinoline (7,4 g, 1 éq) dans 100ml d'HCl (0,5 N)
Acide sulfanilique (8,66 g, 1 éq.) dans 50ml d'eau
Nitrite de sodium (2,8 g, 1,08 éq.) dans 10 ml d'eau
Acide chlorhydrique 35% (10,8 ml, 2,5 éq.) dans 60g de glace
Masse obtenue : 5,5 g (rendement 33%) solide rouge vif
M(m/z) : M-= 331

### 1b) Acide 4-[(E)-(7-amino-2,3-dihydro-1H-indèn-4-yl)diazènyl]-benzènesulfonique

amine (B) : 4 aminoindane (5 g, 1 éq.) dans 40ml d'HCl (0.5N)
Acide sulfanilique (6,51 g, 1 éq.) dans 40ml d'eau
Nitrite de sodium (2,8 g, 1,08 éq.) dans 8 ml d'eau
Acide chlorhydrique 35% (9,5 ml, 2,5 éq.) dans 50g de glace
Masse obtenue : 4,9 g (rendement 41%) solide orange
M(m/z) : M+= 318

### 1 c) Acide 4-[(E)-(4-amino-5,6,7,8-tétrahydronaphthalèn-1-yl)diazényl]-benzènesulfonique

amine (B): 1-amino-5,6,7,8-tétrahydronaphtalène (7,36 g, 1 éq.) dans 50ml d'HCl (0,5 N)
Acide sulfanilique (8,66 g, 1 éq.) dans 50ml d'eau
Nitrite de sodium (3,73 g, 1,08 éq.) dans 10 ml d'eau
Acide chlorhydrique 35% (12,25 ml, 2,5 éq.) dans 60g de glace
Masse obtenue 14,58 g (rendement 88%) solide rouge foncé
M(m/z) : M+ = 332

### Synthèse des Para-phénylènediamines 2,3-substituées (composés 2 selon le schéma ci-dessus)

Dans un hydrogénateur de 0,5 litre, on place respectivement les composés 1a, 1b, 1c, le catalyseur (palladium sur charbon) et l'éthanol.
La réduction se fait sous une pression d'hydrogène d'environ six bars et à une température de 50°C. La réaction est suivie par CCM.
Après filtration du catalyseur sous azote, on coule sur de l'acide chlorhydrique aqueux. On évapore le filtrat à sec sous pression réduite. Le produit 2 est cristallisé dans l'éthanol chlorhydrique et séché à 40°C sous vide et sur potasse.

### 2a) Dichlorhydrate de 1,2,3,4-tétrahydroisoquinoline-5,8-diamine

Réactif 1a : (5 g)
Pd/C (1.2 g)
Ethanol absolu (250 ml)
Masse obtenue 0.33 g(rendement 9%) solide blanc
M(m/z) : M+=164

### 2b) Dichlorhydrate d'Indan-4,7-diamine

Réactif 1b: (4,5 g)
Pd/C (1 g)
Ethanol absolu (250 ml)
Masse obtenue 1,5 g (rendement 42%) solide rose pâle
M(m/z) : M+=149

### 2c) Dichlorhydrate de 5,6,7,8-Tétrahydro-naphthalène-1,4-diamine

Réactif 1c: (5 g)
Pd/C (1 g)
Ethanol absolu (250 ml)
Masse obtenue 0,5 g (rendement 14%) solide blanc
M(m/z) : M+=163

### Synthèse du dichlorhvdrate de 4,7-diaminoindan-2-ol (6) :

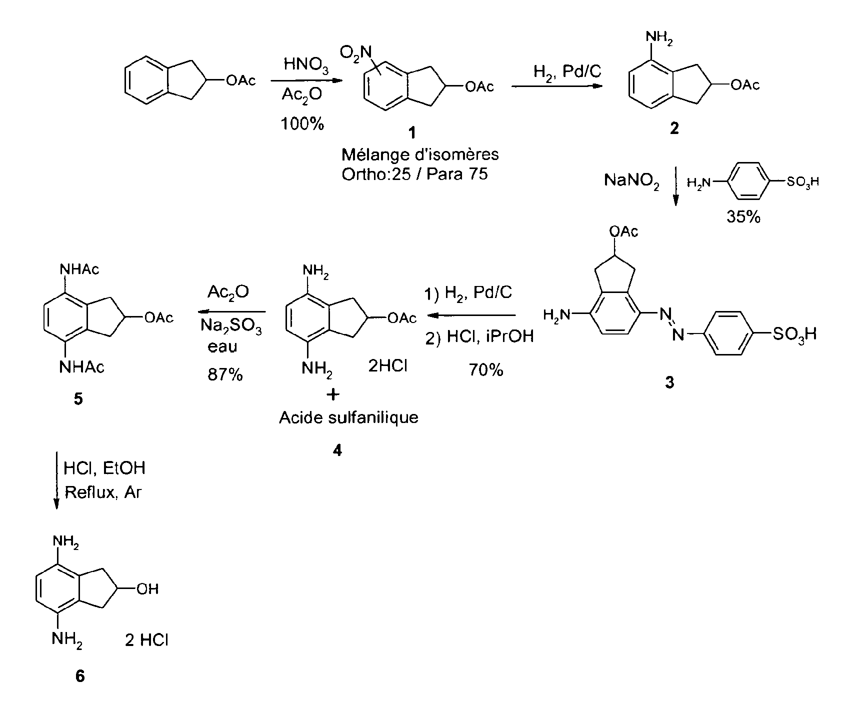

### Synthèse du 4-nitro-2,3-dihydro-1H-indén-2-yl acétate (1)

Dans un tricol muni d'un thermomètre et d'une ampoule de coulée, on mélange 33 g de 2,3-dihydro-1H-inden-2-yl acétate (1 équivalent, 0.188 mol) et 480 ml (27 équivalents, 5.08 mole) d'anhydride acétique. Ce mélange est refroidi à l'aide d'un bain de glace. Un mélange de 120 ml (6.8 équivalents, 1.27 mol) d'anhydride acétique et 39 ml d'acide nitrique (5 équivalents, 0.94 mol), préalablement refroidi, est ajouté goutte à goutte au milieu réactionnel sans dépasser 30°C. la réaction est suivie CCM. Lorsqu'il n'y a plus de produit de départ, on verse le mélange réactionnel dans de la glace et on l'extrait à l'acétate d'éthyle, on lave au carbonate de potassium aqueux puis à l'eau, enfin on sèche sur sulfate de magnésium et on concentre sous vide.
On obtient 38 g (rendement brut=90%) du mélange d'isomères ortho (67%)/para (33%) (huile orange) qui est recristallisé deux fois dans l'éthanol afin d'enrichir l'huile marron finalement obtenue en isomère para. Obtention de 18.8 g (rendement=45%) de mélange d'isomères (ortho 25/para 75) de composé **1.**

### Synthèse du 4-amino-2,3-dihydro-1H-indén-2-yl acétate (2)

Dans un hydrogénateur, on place 24 g (1 équivalent, 0.108 mol) du mélange d'isomères **(1)** obtenu précédemment, 2.5 g de catalyseur (palladium sur charbon) et 1.6 1 d'éthanol.
La réduction se fait sous une pression d'hydrogène de 5 bars jusqu'à disparition du produit de départ (suivi CCM). Après filtration du catalyseur, on évapore le filtrat à sec sous pression réduite. On obtient 23.4 g d'une huile marron qui est purifiée par HPLC préparative. Obtention de 9.8 g (rendement= 47%) de composé **2** pur à 99% en HPLC.

### Synthèse du 4-{(E)-[2-(acétyloxy)-7-amino-2,3-dihydro-1H-indén-4-yl]diazènyl}benzène sulfonique acide (3)

On prépare une solution de 1.62 g nitrite de sodium (0.023 mole, 1 équivalent) dans 6.8 ml d'eau qui est additionnée à froid à 4.07 g d'acide sulfanilique (0.023 mole, 1 équivalent) dissout dans l'eau (couleur orange). La température est maintenue inférieure à 10 °C.
La solution obtenue est immédiatement versée dans un mélange d'acide chlorhydrique concentré (6.24 ml) et de glace (31g).
Le sel de diazonium froid est additionné à 4.5 g (0.023 mole, 1 équivalent) d'aniline (2) en solution dans un mélange d'acide acétique (49.5 ml), d'eau (7.5ml) et de 9.54g d'acétate de sodium. Une couleur orange sang apparaît, le précipite formé est filtré est lavé une fois à l'eau puis à l'éther diisoproylique. Obtention de 1.54g de produit attendu **(3)** (Rendement= 18%).

### Synthèse du dichlorhydrate de 4,7-diamino-2,3-dihydro-1H-indén-2-yl acétate en mélange avec le chlorhydrate d'acide sulfanilique (4)

Dans un hydrogénateur, on place 2.88 g (1 équivalent, 0.0077 mole) du composé (3), 380 mg de catalyseur (palladium sur charbon) et 600 ml de méthanol.
La réduction se fait sous une pression d'hydrogène de 8 bars. La réaction est suivie par CCM. Après filtration du catalyseur sous azote on coule sur de l'isopropanol chlorhydrique. On évapore le filtrat à sec sous pression réduite. On obtient 3.08 g d'une poudre blanche avec un rendement de 82%. Cette poudre est constituée d'un mélange de dihydrochlorhydrate de 4,7-diamino-2,3-dihydro-1H-indén-2-ylacétate (45% en poids) et de l'acide sulfanilique (55% en poids).

### Synthèse du 4,7-bis(acétylamino)-2,3-dihydro-1H-indén-2-yl acétate (5)

Dans un tricol, on mélange 3.08 g (0.006 mole, 1 équivalent) de composé (**4**), 2.4 g (0.019 mole, 3 équivalents) de sulfite de sodium et 30 ml d'eau. On ajoute goutte à goutte à ce mélange réactionnel 1.7 ml (0.019 mole, 3 équivalents) d'anhydride acétique en maintenant la température à environ 5°C. on observe la précipitation d'un solide beige. Le milieu réactionnel est agité jusqu'à revenir à température ambiante. L'agitation est maintenue pendant trois heures. Le produit de départ ayant disparu, on ajoute goutte à goutte 7ml d'ammoniaque à 20% au milieu réactionnel jusqu'à obtention d'un pH=9. Le solide beige est filtré puis séché sous vide. Obtention de 1.59 g (rendement=87%) d'une poudre beige.

### Synthèse du dichlorhydrate de 4,7-diaminoindan-2-ol (6)

Dans un tricol, on mélange 1.59 g de composé **(5),** 10 ml d'éthanol et 10 ml d'acide chlorhydrique à 37%. Le milieu réactionnel est chauffé à reflux. La réaction est suivie par CCM. Lorsque le produit de départ et le produit monoacétylé ont disparu, on laisse revenir à température ambiante. Le précipité est alors filtré, lavé à l'eau puis à l'éther diisopropylique et séché sous vide. Obtention de 944 mg d'une poudre blanche (Rendement= 73 %).
Les spectres RMN du proton et de masse sont conformes à la structure attendue du produit.

### Exemples de formulation

### Exemples 1 à 13 : Composition tinctoriale à partir du dichlorhydrate d'Indan-4,7-diamine (2b)

### - Exemples 1 à 7 : Teinture en milieu acide

Les compositions tinctoriales suivantes sont préparées :

| Exemple | 1 | 2 | 3 | 4 | 5 | 6 | 7 |
|---|---|---|---|---|---|---|---|
| Dichlorhydrate d' Indan-4,7-diamine | 10⁻³ mole | 10⁻³ mole | 10⁻³ mole | 10⁻³ mole | 10⁻³ mole | 10⁻³ mole | 10⁻³ mole |
| Benzène-1,3-diol | 10⁻³ mole | | | | | | |
| 5-Amino-2-méthyl-phénol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Diméthyl-1H-pyrazolo[5,1-c][1,2 ,4]triazole | | | | | 10⁻³ mole | | |
| chlorhydrate de 2-(2,4-Diamino-phénoxy)- éthanol, | | | | | | 10⁻³ mole | |
| chlorhydrate de 3-Amino-2-chloro-6-méthyl-phénol | | | | | | | 10⁻³ mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 1 | 2 | 3 | 4 | 5 | 6 | 7 |
|---|---|---|---|---|---|---|---|
| Nuance observée | orangé | violet-rouge intense | rouge intense | brun rouge intense | rouge | bleu intense | violet intense |

### - Exemples 8 à 13 : teinture en milieu basique

Les compositions tinctoriales suivantes sont préparées :

| Exemple | 8 | 9 | 10 | 11 | 12 | 13 |
|---|---|---|---|---|---|---|
| Dichlorhydrate d'Indan-4,7-diamine | 10⁻³ mole | 10⁻³ mole | 10⁻³ mole | 10⁻³ mole | 10⁻³ mole | 10⁻³ mole |
| 5-Amino-2-méthyl-phénol | 10⁻³ mole | | | | | |
| 1H-Indol-6-ol | | 10⁻³ mole | | | | |
| 2-Amino-pyridin-3-ol | | | 10⁻³ mole | | | |
| 3,6-Diméthyl-1H-pyrazolo[5,1-c][1,2 ,4]triazole | | | | 10⁻³ mole | | |
| chlorhydrate de 2-(2,4-Diamino-phénoxy)-ethanol | | | | | 10⁻³ mole | |
| chlorhydrate de 3-Amino-2-chloro-6-méthyl-phénol | | | | | | 10⁻³ mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (2) pH 9.5 | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH4Cl | 4,32 g |
| Ammoniaque à 20% de NH3 | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 8 | 9 | 10 | 11 | 12 | 13 |
|---|---|---|---|---|---|---|
| Nuance observée | violet-rouge intense | orangé intense | rouge | rouge chromatique | bleu intense | violet intense |

### Exemples 14 à 26 : Composition tinctoriale à partir du Dichlorhydrate de 5,6,7,8-Tétrahydro-naphthalène-1,4-diamine (2c)

### - Exemples 14 à 20 : Teinture en milieu acide

Les compositions tinctoriales suivantes sont préparées

| Exemple | 14 | 15 | 16 | 17 | 18 | 19 | 20 |
|---|---|---|---|---|---|---|---|
| Dichlorhydrate de 5,6,7,8-Tétrahydro-naphthalène-1,4-diamine | 10⁻³ mole | 10⁻³ mole | 10⁻³ mole | 10⁻³ mole | 10⁻³ mole | 10⁻³ mole | 10⁻³ mole |
| Benzène-1,3-diol | 10⁻³ mole | | | | | | |
| 5-Amino-2-méthyl-phénol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Diméthyl-1H-pyrazolo[5,1-c][1,2 ,4]triazole | | | | | 10⁻³ mole | | |
| chlorhydrate de 2-(2,4-Diamino-phénoxy)-éthanol | | | | | | 10⁻³ mole | |
| chlorhydrate de 3-Amino-2-chloro-6-méthyl-phénol | | | | | | | 10⁻³ mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 14 | 15 | 16 | 17 | 18 | 19 | 20 |
|---|---|---|---|---|---|---|---|
| Nuance observée | orangé | violet-rouge intense | rouge intense | brun rouge intense | rouge | bleu intense | violet-bleu intense |

### - Exemples 21 à 26 : teinture en milieu basique

Les compositions tinctoriales suivantes sont préparées :

| Exemple | 21 | 22 | 23 | 24 | 25 | 26 |
|---|---|---|---|---|---|---|
| Dichlorhydrate de 5,6,7,8-Tétrahydronaphthalène-1,4-diamine | 10⁻³ mole | 10⁻³ mole | 10⁻³ mole | 10⁻³ mole | 10⁻³ mole | 10⁻³ mole |
| 5-Amino-2-méthylphénol | 10⁻³ mole | | | | | |
| 1H-Indol-6-ol | | 10⁻³ mole | | | | |
| 2-Amino-pyridin-3-ol | | | 10⁻³ mole | | | |
| 3,6-Diméthyl-1H-pyrazolo[5,1-c][1,2 ,4]triazole | | | | 10⁻³ mole | | |
| chlorhydrate de 2-(2,4-Diamino-phénoxy)-éthanol | | | | | 10⁻³ mole | |
| chlorhydrate de 3-Amino-2-chloro-6-méthyl-phénol | | | | | | 10⁻³ mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (2) pH 9.5 | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 21 | 22 | 23 | 24 | 25 | 26 |
|---|---|---|---|---|---|---|
| Nuance observée | violet intense | orangé | orangé | rouge chromatique | bleu intense | violet-bleu intense |

### Exemples 27 à 33: Composition tinctoriale à partir du Dichlorhvdrate de 1,2,3,4-tétrahydroisoquinoline-5,8-diamine

### Exemples 27 à : Teinture en milieu acide

Les compositions tinctoriales suivantes sont préparées :

| **Exemple** | **27** | **28** | **29** | **30** |
|---|---|---|---|---|
| Dichlorhydrate de 1,2,3,4-tétrahydroisoquinoline-5,8-diamine | 10⁻³ mole | 10⁻³ mole | 10⁻³ mole | 10⁻³ mole |
| 5-Amino-2-méthyl-phénol | 10⁻³ mole | | | |
| 1H-Indol-6-ol | | 10⁻³ mole | | |
| chlorhydrate de 2-(2,4-Diaminophénoxy)-éthanol | | | 10⁻³ mole | |
| chlorhydrate de 3-Amino-2-chloro-6-méthyl-phénol | | | | 10⁻³ mole |
| Support de teinture (2) | (*) | (*) | (*) | (*) |
| Eau déminéralisée q.s.p. | 100g | 100g | 100g | 100g |

| | | | | |
|---|---|---|---|---|
| (*) : support de teinture (1) pH 7 | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 27 | 28 | 29 | 30 |
|---|---|---|---|---|
| Nuance observée | rouge | orangé | gris bleu | violet-rouge |

### - Exemples 31 à 33 : teinture en milieu basique

Les compositions tinctoriales suivantes sont préparées :

| **Exemple** | **31** | **32** | **33** |
|---|---|---|---|
| Dichlorhydrate de 1,2,3,4-tétrahydroisoquinoline-5,8-diamine | 10⁻³ mole | 10⁻³ mole | 10⁻³ mole |
| 5-Amino-2-méthyl-phénol | 10⁻³ mole | | |
| chlorhydrate de 2-(2,4-Diamino-phénoxy)-éthanol | | 10⁻³ mole | |
| chlorhydrate de 3-Amino-2-chloro-6-méthyl-phénol | | | 10⁻³ mole |
| Support de teinture (1) | (*) | (*) | (*) |
| Eau déminéralisée q.s.p. | 100g | 100g | 100g |

| | | | |
|---|---|---|---|
| (*) : support de teinture (2) pH 9.5 | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 31 | 32 | 33 |
|---|---|---|---|
| Nuance observée | rouge | bleu | violet-rouge |

### Exemples 34 à 41 : Composition tinctoriale à partir du dichlorhydrate de 4,7-diaminoindan-2-ol

### - Exemples 34 à 38: Teinture en milieu acide

Les compositions tinctoriales suivantes sont préparées :

| Exemple | 34 | 35 | 36 | 37 | 38 |
|---|---|---|---|---|---|
| dichlorhydrate de 4,7-diaminoindan-2-ol | 10⁻³ mole | 10⁻³ mole | 10⁻³ mole | 10⁻³ mole | 10⁻³ mole |
| 5-Amino-2-méthyl-phénol | 10⁻³ mole | | | | |
| 1H-Indol-6-ol | | 10⁻³ mole | | | |
| 2-Amino-pyridin-3-ol | | | 10⁻³ mole | | |
| 2-(2,4-Diamino-phénoxy)-éthanol,chlorhydrate | | | | 10⁻³ mole | |
| 3-Amino-2-chloro-6-méthylphénol,chlorhydrate | | | | | 10⁻³ mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (1) pH 7 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na2HPO4 | 0,28 g |
| KH2PO4 | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous :

| Exemple | 34 | 35 | 36 | 37 | 38 |
|---|---|---|---|---|---|
| Nuance observée | rouge | orangé | brun rouge | gris bleu intense | violet |

### - Exemples 39 à 41 : teinture en milieu basique

Les compositions tinctoriales suivantes sont préparées :

| Exemple | 39 | 40 | 41 |
|---|---|---|---|
| dichlorhydrate de 4,7-diaminoindan-2-ol | 10⁻³ mole | 10⁻³ mole | 10⁻³ mole |
| 5-Amino-2-méthyl-phénol | 10⁻³ mole | | |
| 2-(2,4-Diamino-phénoxy)-éthanol,chlorhydrate | | 10⁻³ mole | |
| 3-Amino-2-chloro-6-méthyl-phénol,chlorhydrate | | | 10⁻³ mole |
| Support de teinture (2) | (*) | (*) | (*) |
| Eau déminéralisée q.s.p. | 100g | 100g | 100g |

| | | | |
|---|---|---|---|
| (*) : support de teinture (2) pH 9.5 | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH4C1 | 4,32 g |
| Ammoniaque à 20% de NH3 | 2,94 g |

Au moment de l'emploi, chaque composition est mélangée avec un poids égal d'eau oxygénée à 20 volumes (6% en poids). On obtient un pH final de 9,5.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.
Les nuances obtenues figurent dans le tableau ci-dessous :

| Exemple | 39 | 40 | 41 |
|---|---|---|---|
| Nuance observée | rouge | gris bleu | violet |

## Revendications

1. Composé para-phénylènediamine de formule (I), ses solvates et sels d'acide physiologiquement acceptables : 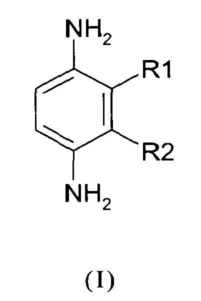 dans laquelle R₁ et R₂ forment ensemble avec les atomes de carbone auxquels ils sont rattachés un cycle à 4, 5, 6 ou 7 chaînons, **caractérisé en ce que** le cycle est soit constitué d'atomes de carbone, soit constitué d'atomes de carbone et d'un ou deux atomes choisis parmi l'oxygène et l'azote, lorsque le cycle comprend deux atomes choisis parmi l'oxygène et l'azote alors ces deux atomes sont non adjacents, le cycle pouvant être substitué,
à l'exception des composés suivants :

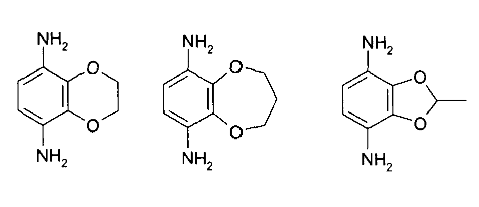

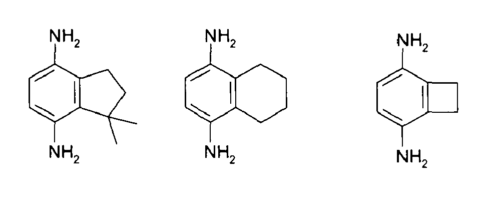

2. Composé de formule (I) selon la revendication 1 **caractérisé par le fait que** R₁ et R₂ forment ensemble avec les atomes de carbone auxquels ils sont rattachés un cycle à 5 ou 6 chaînons.

3. Composé de formule (I) selon la revendication 1 ou 2 **caractérisé par le fait que** le cycle est soit constitué d'atomes de carbone, soit constitué d'atomes de carbone et d'un atome choisi parmi l'oxygène et l'azote.

4. Composé de formule (I) selon l'une quelconque des revendications 1 à 3, **caractérisé par le fait que** le cycle est substitué par un ou plusieurs radicaux identiques ou non choisis parmi les radicaux alkyle linéaires ou ramifiés en C₁-C₆, hydroxyle (-OH), alcoxy en C₁-C₆, amino (-NH₂), (mono ou dialkyle) amino en C₁-C₆, ou aminoalkyle en C₁-C₆.

5. Composé selon la revendication 4 dans lequel le cycle est substitué par 1, 2 ou 3 radicaux choisis parmi les radicaux alkyle en C₁-C₃, hydroxyle (-OH), amino (-NH₂) ou aminoalkyle en C₁-C₃.

6. Composé de formule (I) selon l'une quelconque des revendications précédentes dans lequel le cycle est un cycle carboné à 5 ou 6 chaînons.

7. Composé de formule (I) selon l'une quelconque des revendications 1 à 6 dans lequel le cycle est un cycle à 5 ou 6 chainons renfermant un radical divalent -NH- ou -NMe- .

8. Composé de formule (I) selon l'une quelconque des revendications précédentes, choisi parmi les composés suivants ou l'un de leurs sels ou solvates physiologiquement acceptables
| | |
|---|---|
| 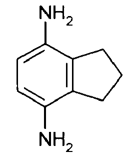 | **Indan-4,7-diamine** |
| 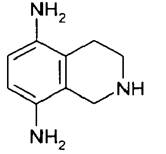 | **1,2,3,4-Tétrahydro-isoquinoline-5,8-diamine** |
| 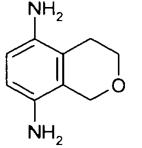 | **Isochroman-5,8-diamine** |
| 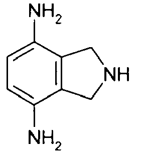 | **2,3-Dihydro-1H-isoindole-4,7-diamine** |
| 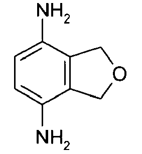 | **1,3-Dihydro-isobenzofuran-4,7-diamine** |
| 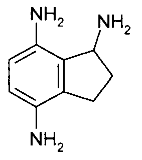 | **Indan-1,4,7-triamine** |
| 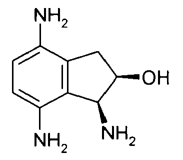 | **1,4,7-Triamino-indan-2-ol** |
| 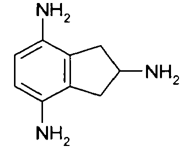 | **Indan-2,4,7-triamine** |
| 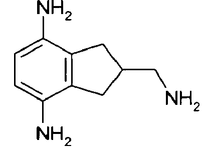 | **2-Aminométhyl-indan-4,7-diamine** |
| 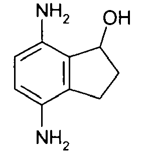 | **4,7-Diamino-indan-1-ol** |
| 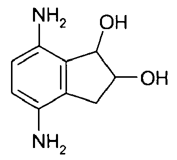 | **4,7-Diamino-indan-1,2-diol** |
| 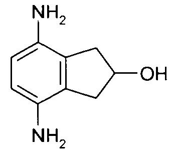 | **4,7-Diamino-indan-2-ol** |
| 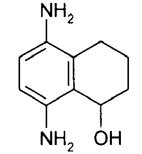 | **5,8-Diamino-1,2,3,4-tétrahydronaphthalèn-1-ol** |
| 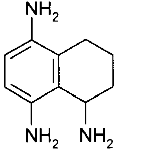 | **5,6,7,8-Tétrahydro-naphthalène-1,4, 5-triamine** |
| 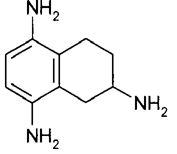 | **5,6,7,8-Tétrahydro-naphthalène-1,4, 6-triamine** |
| 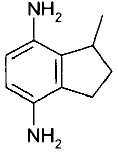 | **1-Méthyl-indan-4,7-diamine** |
| 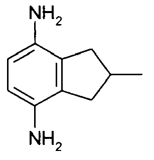 | **2-Méthyl-indan-4,7-diamine** |
| 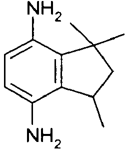 | **1,1,3-Triméthyl-indan-4,7-diamine** |
| 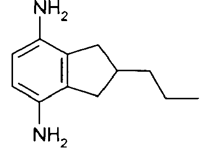 | **2-Propyl-indan-4,7-diamine** |
| 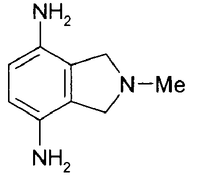 | **2-méthylisoindoline-4,7-diamine** |

9. Composé de formule (I) selon l'une quelconque des revendications précédentes choisi parmi l'indan-4,7-diamine, le 1,2,3,4-tétrahydro-isoquinoline-5,8-diamine, le 2,3-dihydro-1H-isoindole-4,7-diamine, le 2-méthylisoindoline-4,7-diamine, l'indan-2,4,7-triamine, le 2-aminométhyl-indan-4,7-diamine, le 4,7-diamino-indan-2-ol, la 2-méthyl-indan-4,7-diamine et le 2-propyl-indan-4,7-diamine ou l'un de leurs sels ou solvates physiologiquement acceptables.

10. Procédé de synthèse d'un composé para-phénylènediamine de formule (I) selon l'une quelconque des revendications précédentes **caractérisé en ce qu'**il comprend une étape de réduction du composé intermédiaire de formule (II) correspondant : 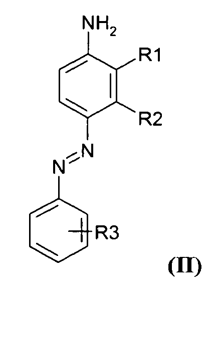 dans laquelle R₁ et R₂ forment ensemble avec les atomes de carbone auxquels ils sont rattachés un cycle à 4, 5, 6 ou 7 chaînons, **caractérisé en ce que** le cycle est soit constitué d'atomes de carbone, soit constitué d'atomes de carbone et d'un ou deux atomes choisis parmi l'oxygène et l'azote, lorsque le cycle comprend deux atomes choisis parmi l'oxygène et l'azote alors ces deux atomes sont non adjacents, le cycle pouvant être substitué ; R₃ est choisi dans le groupe formé par un atome d'hydrogène, un groupement sulfonique, les radicaux alkyle linéaires ou ramifiés en C₁-C₄,
à l'exception des composés suivants :

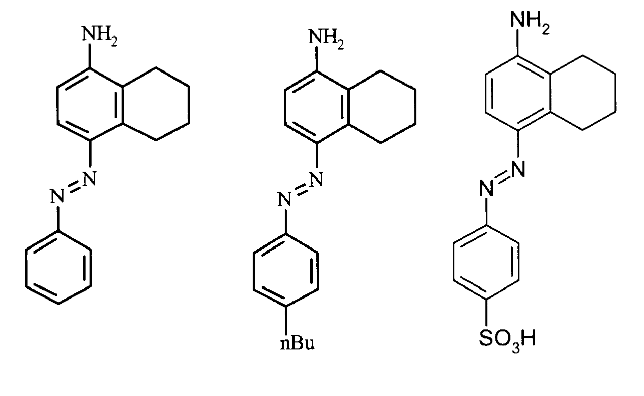

11. Composé intermédiaire de formule II : 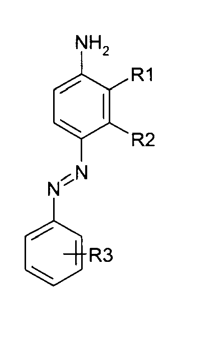 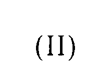 dans laquelle R₁ et R₂ forment ensemble avec les atomes de carbone auxquels ils sont rattachés un cycle à 4, 5, 6 ou 7 chaînons, **caractérisé en ce que** le cycle est soit constitué d'atomes de carbone, soit constitué d'atomes de carbone et d'un ou deux atomes choisis parmi l'oxygène et l'azote, lorsque le cycle comprend deux atomes choisis parmi l'oxygène et l'azote alors ces deux atomes sont non adjacents, le cycle étant éventuellement substitué ; R₃ est choisi dans le groupe formé par un atome d'hydrogène, un groupement sulfonique, les radicaux alkyle linéaires ou ramifiés en C₁-C₄,
à l'exception des composés suivants :

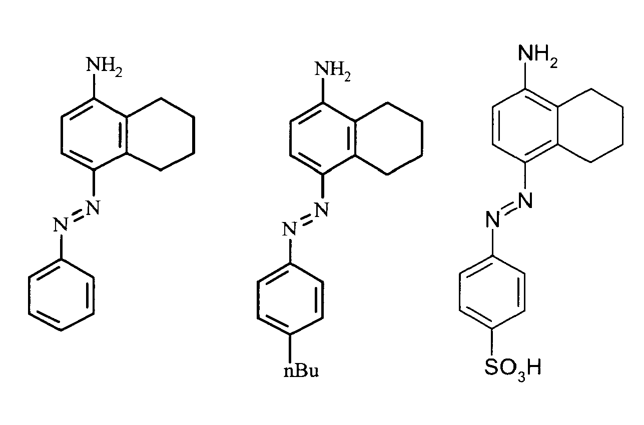

12. Composition pour la teinture d'oxydation des fibres kératiniques, **caractérisée par le fait qu'**elle contient, dans un milieu approprié pour la teinture, au moins un composé para-phénylènediamine de formule (I), ses solvates et sels d'acide physiologiquement acceptables: 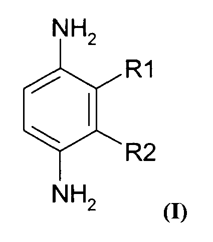 dans laquelle R₁ et R₂ forment ensemble avec les atomes de carbone auxquels ils sont rattachés un cycle à 4, 5, 6 ou 7 chaînons, **caractérisé en ce que** le cycle est soit constitué d'atomes de carbone, soit constitué d'atomes de carbone et d'un ou deux atomes choisis parmi l'oxygène et l'azote, lorsque le cycle comprend deux atomes choisis parmi l'oxygène et l'azote alors ces deux atomes sont non adjacents, le cycle étant éventuellement substitué, à l'exception des composés suivants :

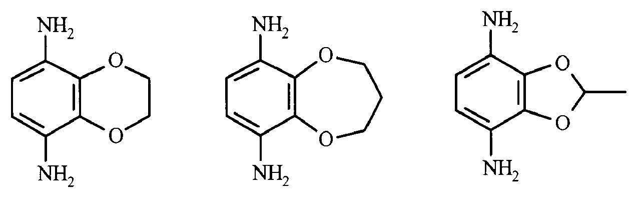

13. Composition selon la revendication précédente dans laquelle le composé para-phénylènediamine de formule (I) est tel que le cycle formé par R₁ et R₂ avec les atomes de carbone auxquels ils sont rattachés est un cycle carboné ou azoté.

14. Composition selon la revendication 12 ou 13, **caractérisée par le fait que** le composé para-phénylènediamine de formule (I) est choisi parmi les composés suivants : l'indan-4,7-diamine, le 5,6,7,8-tétrahydro-naphtalène-1,4-diamine, le 1,2,3,4-tétrahydro-isoquinoline-5,8-diamine, l'isochroman-5,8-diamine, le 2,3-dihydro-1H-isoindole-4,7-diamine, la 2-méthylisoindoline-4,7-diamine, le 1,3-dihydro-isobenzofuran-4,7-diamine, l'indan-1,4,7-triamine, le 1,4,7-triamino-indan-2-ol, l'indan-2,4,7-triamine, le 2-aminométhyl-indan-4,7-diamine, le 4,7-diamino-indan-1-ol, le 4,7-diamino-indan-1,2-diol, le 4,7-diamino-indan-2-ol, le 5,8-diamino-1,2,3,4-tétrahydro-naphtalèn-1-ol, le 5,6,7,8-tétrahydro-naphtalène-1,4,5-triamine, le 5,6,7,8-tétrahydro-naphtalène-1,4,6-triamine, le 1-méthyl-indan-4,7-diamine, le 2-méthyl-indan-4,7-diamine, le 1,1,3-triméthyl-indan-4,7-diamine et le 2-propyl-indan-4,7-diamine ou l'un de leurs sels ou solvates physiologiquement acceptables.

15. Composition selon la revendication 14, dans laquelle le composé para-phénylènediamine de formule (I) est choisi parmi l'indan-4,7-diamine, le 5,6,7,8-tétrahydro-naphtalène-1,4-diamine, le 1,2,3,4-tétrahydro-isoquinoline-5,8-diamine, le 2.3-dihydro-1H-isoindole-4,7-diamine, le 2-méthylisoindoline-4,7-diamine, l'indan-2,4,7-triamine, le 2-aminométhyl-indan-4,7-diamine, le 4,7-diamino-indan-2-ol, la 2-méthyl-indan-4,7-diamine et le 2-propyl-indan-4,7-diamine ou l'un de leurs sels ou solvates physiologiquement acceptables.

16. Composition selon l'une quelconque des revendications 12 à 15 **caractérisée par le fait qu'**elle contient de 0,001 à 10% en poids d'au moins un composé para-phénylènediamine de formule (I), ses sels ou ses solvates.

17. Composition selon la revendication 16 **caractérisée par le fait qu'**elle contient de 0,05 à 6% en poids, et de préférence 0,1 à 3% en poids, d'au moins un composé para-phénylènediamine de formule (I), ses sels ou ses solvates.

18. Composition selon l'une quelconque des revendications 12 à 17, **caractérisée par le fait que** le milieu approprié pour la teinture est constitué par de l'eau ou par un mélange d'eau et d'au moins un solvant organique choisi parmi les alcanols inférieurs en C₁-C₄, les polyols et éthers de polyols, les alcools aromatiques, et leurs mélanges.

19. Composition selon l'une quelconque des revendications 12 à 18, **caractérisée par le fait qu'**elle présente un pH compris entre 3 et 12.

20. Composition selon l'une quelconque des revendications 12 à 19 **caractérisée par le fait qu'**elle contient au moins une base d'oxydation additionnelle choisie parmi les para-phénylènediamines différentes des para-phénylènediamines de formule (I), les bis-phénylalkylènediamines, les para-aminophénols, les ortho-aminophénols, des bases hétérocycliques et leurs sels d'addition avec un acide.

21. Composition selon la revendication 20, **caractérisée par le fait que** la ou les bases d'oxydation additionnelles représentent de 0,0005 à 12% en poids du poids total de la composition tinctoriale.

22. Composition selon l'une quelconque des revendications 12 à 21 **caractérisée par le fait qu'**elle contient au moins un coupleur et/ou au moins un colorant direct.

23. Composition selon la revendication 22 **caractérisée par le fait que** le ou les coupleurs sont choisis parmi les méta-phénylènediamines, les méta-aminophénols, les méta-diphénols, les dérivés mono- ou polyhydroxylés du naphtalène et les coupleurs hétérocycliques et leurs sels d'addition avec un acide.

24. Composition selon l'une quelconque des revendications 22 ou 23 **caractérisée par le fait que** le ou les coupleurs représentent de 0,0001 à 10% en poids du poids total de la composition tinctoriale.

25. Composition selon l'une quelconque des revendications 12 à 24 , **caractérisée par le fait que** les sels d'addition avec un acide sont choisis parmi les sels des acides suivants : chlorhydrique, bromhydrique, sulfurique, acétique, lactique, tartrique, citrique, méthanesulfonique, para-toluènesulfonique, benzènesulfonique, phosphorique ou succinique.

26. Procédé de teinture des fibres kératiniques et en particulier des fibres kératiniques humaines telles que les cheveux, **caractérisé par le fait que** l'on applique sur ces fibres au moins une composition tinctoriale telle que définie à l'une quelconque des revendications 12 à 25 pendant un temps suffisant pour développer la coloration désirée, soit à l'air, soit à l'aide d'un agent oxydant, éventuellement en présence de catalyseurs d'oxydation.

27. Procédé selon la revendication 26 **caractérisé par le fait que** la coloration est révélée au seul contact de l'oxygène de l'air.

28. Procédé selon la revendication 26 **caractérisé par le fait que** l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

29. Procédé selon la revendication 26, **caractérisé par le fait que** l'agent oxydant est choisi parmi le peroxyde d'hydrogène, le peroxyde d'urée, les bromates de métaux alcalins, les persels tels que les perborates et persulfates.

30. Dispositif à plusieurs compartiments, ou "kit" de teinture à plusieurs compartiments, dont un premier compartiment renferme une composition tinctoriale telle que définie à l'une quelconque des revendications 12 à 25 et un deuxième compartiment renferme une composition oxydante.
